(19) Europäisches Patentamt
European Patent Office
Office européen des brevets

(11) EP 0 629 394 B1

(12) **FASCICULE DE BREVET EUROPEEN**

(45) Date de publication et mention de la délivrance du brevet: **12.08.1998 Bulletin 1998/33**

(51) Int. Cl.[6]: **A61K 7/02**, A61K 7/035, A61K 7/48

(21) Numéro de dépôt: **94401156.8**

(22) Date de dépôt: **25.05.1994**

(54) **Utilisation d'un composé organofluoré hydrocarboné, comme liant dans des compositions cosmétiques sous forme de poudre et composition en comportant**

Verwendung von Organofluorkohlenwasserstoffverbindungen als Bindemittel in kosmetischen Puderzusammensetzungen und diese enthaltende Zusammensetzungen

Use of an organofluorinated hydrocarbon as binder in powder cosmetic compositions and composition containing it

(84) Etats contractants désignés:
**DE ES FR GB IT**

(30) Priorité: **26.05.1993 FR 9306326**

(43) Date de publication de la demande:
**21.12.1994 Bulletin 1994/51**

(73) Titulaire: **L'OREAL**
**75008 Paris (FR)**

(72) Inventeurs:
- **Mondon-Rossignol, Sylvie**
  **F-75014 Paris (FR)**
- **Defossez, Béatrice**
  **F-75020 Paris (FR)**

(74) Mandataire: **Casalonga, Axel**
**BUREAU D.A. CASALONGA - JOSSE**
**Morassistrasse 8**
**80469 München (DE)**

(56) Documents cités:
**EP-A- 0 469 602** **EP-A- 0 545 786**
**WO-A-93/11103** **US-A- 3 632 744**

- **DATABASE WPI Week 9324, Derwent Publications Ltd., London, GB; AN 93-190862 & JP-A-5 115 769 (KAO CORP) 14 Mai 1993**
- **DATABASE WPI Week 9325, Derwent Publications Ltd., London, GB; AN 93-200416 & JP-A-5 124 933 (KAO CORP) 21 Mai 1993**
- **PATENT ABSTRACTS OF JAPAN vol. 12, no. 198 (C-502) 8 Juin 1988 & JP-A-63 002 916 (KANEBO LTD) 7 Janvier 1988**

EP 0 629 394 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne l'utilisation de composés organofluorés hydrocarbonés à titre de liant dans la préparation de compositions cosmétiques sous forme de poudre, ainsi qu'une composition cosmétique sous forme de poudre anhydre comportant de tels composés.

Dans le domaine des compositions cosmétiques poudreuses, sous forme de poudres compactes ou libres pour le maquillage du visage ou encore de poudres corporelles, il est connu d'utiliser, d'une part, une phase particulaire comportant notamment pigments et charges et, d'autre part, une phase grasse comportant des liants comme des corps gras, destinés à conférer au produit fini une certaine densité, à donner une certaine cohésion aux particules minérales et/ou organiques intervenant comme charges, à donner douceur et émollience au produit de maquillage et à favoriser son adhérence sur la peau.

Pour obtenir une cohésion appropriée, on peut utiliser un mélange d'huiles minérales et végétales associées à des esters et/ou des alcools gras. L'utilisation de ces huiles minérales et végétales peut cependant mener à des produits cosmétiques manquant de douceur.

Par ailleurs, pour pallier à cet inconvénient du manque de douceur, on peut utiliser des huiles de silicones ou des mélanges de résine, cire, huile ou gomme de silicone. L'emploi de produits à base de silicones est cependant limité par leur incompatibilité avec certaines autres huiles et notamment leur insolubilité dans certaines autres huiles.

Enfin, certaines huiles perfluorées, en particulier les perfluoropolyéthers sont bien connus comme apportant de la douceur aux compositions cosmétiques, mais leur usage est limité du fait de leur insolubilité dans les huiles hydrocarbonées (minérales, végétales ou animales) usuelles ou les huiles de silicones, cette insolubilité entraînant des problèmes d'instabilité de la composition cosmétique.

Parmi les perfluoropolyéthers connus, les composés du type "FOMBLIN" et notamment le produit "FOMBLIN HC25" commercialisé par la Société MONTEDISON, présente en outre l'inconvénient du mauvais développement de la phase pigmentaire : la teinte apportée par le pigment est relativement peu intense, l'emploi du FOMBLIN provoquant un effet blanc.

Ainsi, pour obtenir la teinte désirée par rapport à un témoin, par l'utilisation des FOMBLINS, il s'est avéré nécessaire d'augmenter le taux de la phase pigmentaire de 25 ou même 60%; en plus du surcoût que cette augmentation induit, elle a pour conséquence une diminution du taux de la phase des charges minérales et/ou organiques dans la phase particulaire, et la formulation cosmétique en est plus difficile à mettre en oeuvre; de plus, la palette de coloris est limitée.

Le document EP-A-0 469 602, par exemple, décrit des compositions cosmétiques sous la forme de poudre qui comportent :

(A) une poudre, par exemple un pigment, traitée avec un composé fluoré, de préférence un polyfluoroacrylphosphate ;
(B) un composé organique liquide perfluoré, de préférence un perfluoropolyéther tel que les produits commercialisés sous les marques FOMBLIN HC-O4 et HC-25 ; et, éventuellement
(C) une huile de silicone.

La demanderesse a maintenant mis au point des compositions cosmétiques sous forme de poudre où le liant comporte un composé organofluoré hydrocarboné.

Ces compositions cosmétiques sous forme de poudre ont à la fois une bonne cohésion et d'excellentes propriétés cosmétiques : les huiles organofluorées hydrocarbonées confèrent de bonnes propriétés de lubrification, une grande douceur, une bonne aptitude à l'étalement, un aspect lisse et homogène des compacts en surface ainsi qu'une compatibilité convenable, voire totale avec des huiles hydrocarbonées ou des huiles de silicones, ainsi qu'un bon développement des pigments.

Pour les composes organofluorés hydrocarbonés, on définit le taux de substitution des atomes d'hydrogène par des atomes de fluor, sous la forme du rapport : nombre d'atomes de fluor/(nombre d'atomes de fluor + nombre d'atomes d'hydrogène) où seuls les atomes d'hydrogène liés aux atomes de carbone du squelette sont pris en compte.

Ainsi, la présente invention concerne l'utilisation de composés organofluorés hydrocarbonés comme liant dans des compositions cosmétiques sous forme de poudre; les composés organofluorés hydrocarbonés ont un taux de substitution des atomes d'hydrogène liés aux atomes de carbone par des atomes de fluor compris entre 0,5 et 95%, et de préférence 10 à 80%.

Selon l'invention, les composés peuvent être utilisés dans des proportions comprises entre 0,1 et 35% en poids par rapport au poids de la composition cosmétique sous forme de poudre.

Les composés organofluorés hydrocarbonés de l'invention ont la formule (I) suivante :

$$(R_F)_x - (A)_y - (R_H)_z \qquad (I)$$

dans laquelle :

x représente 1, 2 ou 3,
y représente 0 ou 1,
z représente 0, 1, 2 ou 3,
à la condition que y et z ne soient pas simultanément nuls et que lorsque z est 0, x est 2 ou 3.

$R_F$ représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,
$R_H$ représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote,
A représente un radical di, tri ou tétravalent tel que $>C<$ , $>CH-$, $-N<$ , $-CO-N<$ , $-SO_2N<$ ,

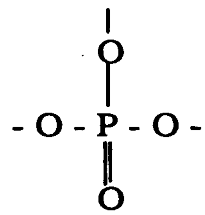

les structures cycliques, aliphatiques ou aromatiques ou les insaturations éthyléniques.

Par l'expression "fonctionnalisé", on entend selon l'invention, une substitution du squelette intercalaire, terminale ou pendante, par au moins un groupement organique fonctionnel comme une fonction alcool, thiol, acide, carbonyle, sulfoxyde, ester, amide, amine, phosphate, éthylénique, acétylénique, et énamine ou sulfonamide. Par insaturation éthylénique, on entend par exemple
$>C = C<$ , $>C = CH-$ ou $-CH = CH-$.

De préférence, $R_H$ représente un radical alkyle linéaire ou ramifié en $C_1$-$C_{22}$ ou un mélange de radicaux alkyle linéaires ou ramifiés en $C_1$-$C_{22}$, un radical aryle en $C_6$-$C_{10}$ ou un radical aralkyle en $C_7$-$C_{15}$.

De préférence, $R_F$ représente un radical perfluoroalkyle ayant de 4 à 22 atomes de carbone.

A titre illustratif, on peut citer les composés possédant des groupes perfluorocarbures et des groupes hydrocarbures, le nombre total de carbone étant compris entre 10 et 30, le nombre d'atomes de carbone des groupes hydrocarbures étant égal ou supérieur à deux fois le nombre d'atomes de carbone des groupes perfluorocarbures, tels qu'ils sont décrits dans le document JP 63-002916.

De même, à titre illustratif, on peut citer les composés organofluorés hydrocarbonés décrits dans la demande de brevet PCT/FR-92/01140 et dont la structure générale est définie par la formule (II) suivante :

$$R_1 - (CH_2)_n - X - [C_3H_5(OH)] - (Y)_x - R_2 \qquad (II)$$

dans laquelle $C_3H_5(OH)$ représente les structures :

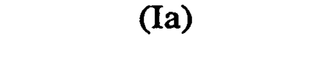

ou

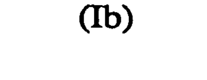

(Ia) (Ib)

$R_1$ représente un radical alkyle perfluoré en $C_4$-$C_{20}$ ou un mélange de radicaux alkyle perfluorés en $C_4$-$C_{20}$;
$R_2$ représente un radical alkyle linéaire ou ramifié en $C_1$-$C_{22}$ ou un mélange de radicaux alkyle linéaires ou ramifiés

en $C_1$-$C_{22}$ ou un radical aryle en $C_6$-$C_{10}$ ou aralkyle en $C_7$-$C_{15}$;
n est compris entre 0 et 4;
X et Y représentent O, S,

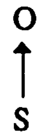 ou 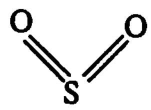

x représente O ou 1 ;
sous réserve que lorsque X est S,

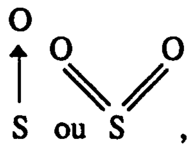 ,

Y représente O .

Ces composés peuvent être préparés en mettant en oeuvre la réaction d'un composé fluoré à hydrogène acide de formule :

$$R_1 - (CH_2)_n - X - H \qquad (III)$$

avec un époxyde de formule :

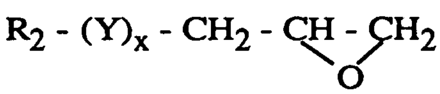 (IV)

ou la réaction d'un composé hydrocarboné à hydrogène acide de formule :

$$R_2 - (Y)_x - H \qquad (V)$$

avec un époxyde fluoré de formule :

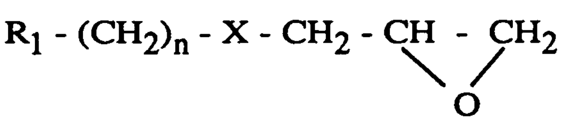 (VI)

en présence d'un composé basique ou acide jouant le rôle de réactif ou de catalyseur, pour obtenir le composé de formule (II) correspondant, les substituants $R_F$, $R_H$, n et x ayant dans les formules ci-dessus la même signification que dans la formule (II) et X et Y désignant O ou S, sous réserve que lorsque X est S, Y est O,
et en oxydant éventuellement la fonction mercaptan en sulfoxyde ou sulfone avec un oxydant, par exemple de l'eau oxygénée en présence d'acide et la récupération du composé obtenu.

Comme exemples de composés de formule (II), on peut citer:

le 1-(2'-F-hexyléthylthio)3-(2''-éthylhexyloxy)2-propanol,
le 1-(2'-F-octyléthylthio)3-(2''-éthylhexyloxy)-2-propanol,
le 1-(2'-F-octyléthylthio)3-butyloxy-2-propanol,

le 1-(2'-F-octyléthylthio)3-phénoxy-2-propanol,
le 1-(2'-F-hexyléthylthio)3-dodécyloxy-2-propanol,
le 1-(2'-F-hexyléthylthio)2-décanol,
le 1-(2'-F-hexyléthylthio)2-hexanol,
le 1-(2'-F-octyléthylthio)2-hexanol, et
le 1-(2'-F-hexyléthyloxy)-3-(2"-éthylhexyloxy)-2-propanol.

Par ailleurs, on peut citer, également, les produits vendus sous la dénomination "NOFABLE FO" par la Société NIPPON OIL & CO, ayant la formule suivante :

$$C_nF_{2n+1}-(CH_2)_p-O-\overset{\overset{\displaystyle O}{\|}}{C}-(CH_2)_7-CH=CH-(CH_2)_7-CH_3 \qquad (VII)$$

dans laquelle n est un nombre entier égal à 6 ou 8 et p est 1 ou 2.

On peut en outre citer les composés décrits notamment par B. Escoula, dans Synthetic Communications, 15(1), 35-38 (1985), de formule (VIII) :

$$R-CH=CH-CH_2-C_nF_{2n+1} \qquad (VIII)$$

dans laquelle :

R désigne un radical butyle ou phényle, et
n est 4, 6 ou 8.

De préférence, selon l'invention, les composés organofluorés hydrocarbonés utilisés sont sous forme d'huile ou de cire.

Le liant selon l'invention est constitué des composés organofluorés hydrocarbonés décrits ci-dessus qui peuvent cependant être associés à des huiles ou des cires hydrocarbonées ou siliconées. Ces cires ou huiles peuvent être choisies parmi :

- les huiles minérales telles que l'huile de paraffine, l'huile de vaseline et les huiles minérales ayant un point d'ébullition compris entre 310 et 410°C;
- les huiles d'origine animale telles que le perhydrosqualène;
- les huiles végétales telles que l'huile d'amande douce, l'huile de calophyllum, l'huile de palme, l'huile d'avocat, l'huile de jojoba, l'huile d'olive, l'huile de ricin, les huiles de germes de céréales telles que l'huile de germes de blé et l'huile de maïs;
- les esters de synthèse tels que l'huile de Purcelin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylèneglycol, l'adipate de diisopropyle;
- les huiles de silicones, volatiles ou non.

Les cires peuvent être d'origine végétale, animale, minérale ou synthétique, telles que la cire de Carnauba, la cire de Candellila, la cire d'abeilles, la cire de baleine, la cire de lanoline ou la cire microcristalline.

Parmi les cires, on peut également noter les cires de polyéthylène, de préférence les homopolymères.

Les composés organofluorés hydrocarbonés de l'invention et les autres corps gras éventuels constituent la phase grasse de la composition, phase qui peut contenir d'autres composés usuellement utilisés dans le domaine cosmétique, et notamment des filtres solaires, des antioxydants, des conservateurs et des principes actifs lipophiles.

La phase grasse de la composition peut également comporter des agents de consistance tels que le polybutène, le polyalkylèneglycol, l'isoprèneglycol, le glycérol ou le sorbitol.

Le poids total de la phase grasse est compris entre 0,1 et 35% du poids total de la composition.

Selon l'invention, la phase particulaire est constituée de charges et/ou de pigments usuellement utilisés dans des compositions cosmétiques sous forme de poudre et peut constituer de 65 à 99,9% du poids total de la composition, le complément étant constitué par la phase grasse; les pigments peuvent représenter jusqu'à 80% en poids de la composition finale.

Les pigments sont choisis parmi les pigments minéraux et/ou organiques et/ou les pigments nacrés.

Parmi les pigments minéraux, on peut citer, à titre d'exemple :

- le dioxyde de titane (rutile ou anatase), éventuellement traité en surface, et codifié dans le Color Index sous la référence CI 77891;
- les oxydes de fer noir, jaune, rouge et grun, codifiés sous les références CI 77499, 77492, 77491;
- le violet de manganèse (CI 77742);
- le violet d'outremer (CI 77007);
- le bleu d'outremer (CI 77007);
- l'oxyde de chrome (CI 77288);
- l'oxyde de chrome hydraté (CI 77289); et
- le bleu ferrique (CI 77510).

Parmi les pigments organiques, on peut citer, en particulier, les pigments :

- D & C red n° 3 (CI 45430:1)
- D & C red n° 6 (CI 15850:2)
- D & C red n° 7 (CI 15850:1)
- D & C red n° 9 (CI 15585:1)
- D & C red n° 13 (CI 15630:3)
- D & C red n° 19 (CI 45170)
- D & C red n° 21 (CI 45380:2)
- D & C red n° 27 (CI 45410:1)
- D & C red n° 30 (CI 73360)
- D & C red n° 36 (CI 12085),
- le noir de carbone (CI 77266) et les laques à base de carmin de cochenille (CI 75470).

Les pigments nacrés peuvent être choisis notamment parmi les pigments nacrés blancs, tels que le mica recouvert d'oxyde de titane ou d'oxychlorure de bismuth. On peut utiliser également des pigments nacrés colorés, tels que le mica titane coloré avec des oxydes de fer, le mica titane coloré avec du bleu ferrique ou de l'oxyde de chrome, le mica titane coloré avec un pigment organique du type précité, ainsi que des pigments nacrés à base d'oxychlorure de bismuth.

Les charges sont choisies notamment parmi :

- le talc, qui est un silicate de magnésium hydraté, qui peut être utilisé sous forme de particules généralement de dimensions inférieures à 40 µm; le talc possède des propriétés absorbantes de l'humidité et est utilisé surtout en raison de son toucher onctueux;
- les micas, qui sont des aluminosilicates de compositions variées, qui se présentent sous la forme d'écailles ayant des dimensions de 2 à 200 µm, de préférence de 5 à 70 µm et une épaisseur de 0,1 à 5 µm, de préférence de 0,2 à 3 µm. Les micas peuvent être d'origine naturelle (par exemple muscovite, margarite, roscoelithe, lipidolithe, biotite) ou d'origine synthétique;
- l'amidon, modifié ou non, en particulier l'amidon de riz;
- la silice;
- l'alumine;
- le nitrure de bore;
- le kaolin, qui est un silicate d'aluminium hydraté, qui se présente sous la forme de particules de forme isotrope;
- les oxydes de zinc et de titane dont on peut aussi utiliser les formes nanopigmentaires;
- l'oxychlorure de bismuth;
- le carbonate de calcium précipité, notamment sous forme de particules de dimensions inférieures à 10 µm environ;
- le carbonate ou l'hydrocarbonate de magnésium;
- des savons métalliques dérivés d'acides organiques carboxyliques, ayant de 8 à 22 atomes de carbone, de préférence de 12 à 18 atomes de carbone, par exemple le stéarate de zinc, de magnésium ou de lithium, le laurate de zinc, le myristate de magnésium, notamment. Ces savons se présentent généralement sous la forme de particules ayant des dimensions inférieures à 10 µm;
- les poudres de polymères (ou copolymères) synthétiques choisies parmi le polyéthylène et ses dérivés, par exemple le polytétrafluoroéthylène ou le polystyrène, les polyacrylates, les polyméthacrylates, les polyesters ou les polyamides, notamment, et par exemple la poudre de nylon;
- les poudres sous forme de microsphères creuses en matériau synthétique thermoplastique, dont la partie creuse contient un gaz.

Les microsphères creuses sont préparées selon les procédés connus, tels que ceux décrits dans le brevet US 3.615.972 ou la demande de brevet européen n° 0056 219.

Ces microsphères peuvent être réalisées en tous matériaux thermoplastiques non toxiques et non irritants. Ces matériaux peuvent être par exemple des polymères ou copolymères de dérivés éthyléniques, par exemple le polyéthylène, le polystyrène, le copolymère chlorure de vinyle-acrylonitrile, des polyesters, des polymères urée-formaldéhyde, des copolymères de chlorure de vinylidène, par exemple le copolymère chlorure de vinylidène-acrylonitrile.

Les charges peuvent représenter jusqu'à 95% du poids total de la composition de l'invention.

Selon l'invention, en outre, des substances comme des acides aminés, des savons métalliques, des silicones ou des composés fluorés, peuvent enrober les pigments et les charges afin de modifier leur état de surface.

La présente invention concerne également une composition cosmétique sous forme de poudre comportant une phase grasse et une phase particulaire comportant pigments et/ou charges, caractérisée en ce qu'elle comporte 98,99 à 65% en poids de phases particulaires et 1,01 à 35% en poids d'au moins un composé organofluoré hydrocarboné ayant un taux de substitution des atomes d'hydrogène liés aux atomes de carbone par des atomes de fluor compris entre 0,5 et 95%.

De préférence, les compositions de l'invention comportent 3 à 15% en poids de composé organofluoré hydrocarboné, par rapport au poids total de la composition.

Les compositions de l'invention se présentent notamment sous la forme de fards à paupières, de fards à joues, de poudres compactes ou libres pour le maquillage du visage, ou sous forme de poudres corporelles.

Selon que les poudres sont compactées ou non, elles sont préparées selon l'un des deux procédés décrits ci-dessous.

Pour préparer des poudres compactées, dans un premier temps, on mélange les pigments et/ou les charges, ainsi que les additifs poudreux, puis on ajoute le liant et éventuellement les agents de consistance, ainsi que d'autres ingrédients éventuels, et le tout est mélangé et/ou éventuellement broyé.

On pourra éventuellement chauffer le liant si nécessaire.

Le mélange est ensuite compacté à l'aide d'une presse dans des coupelles métalliques.

Pour préparer des poudres libres, les pigments et/ou charges ainsi que les additifs poudreux sont mélangés, puis on ajoute le liant et éventuellement les agents de consistance ainsi que d'autres ingrédients éventuels et le tout est mélangé et éventuellement broyé.

Le cas échéant, le liant est chauffé. Si on le souhaite, le mélange peut être tamisé avant un conditionnement dans un récepteur convenable.

**EXEMPLES DE PREPARATION**

EXEMPLE I

**1-(2'-F-hexyléthylthio) 3-(2"-éthylhexyloxy)-2-propanol**

A la température de 25°C, sous agitation et sous courant d'azote, on ajoute à 152 g de 2-F-hexyléthanethiol, 3,6 g d'une solution méthanolique de méthylate de sodium (environ 30% - 5,54 meq $g^{-1}$) en une minute.

Le mélange est chauffé à 70°C. On évapore sous vide le méthanol présent dans le milieu.

Le 2-éthylhexylglycidyléther (74,4 g) est ensuite ajouté goutte-à-goutte en une heure. On maintient la température du mélange entre 60 et 70°C au cours de l'addition de l'époxyde.

A la fin de l'addition, la température est amenée à 25°C.

Le mélange est neutralisé à l'aide de 20 ml de HCl Normal.

Le 1-(2'-F-hexyléthylthio)3-(2"-éthylhexyloxy)2-propanol est séparé par distillation : Eb = 141°C/66,5 Pa.

On obtient 175 g (77%) d'une huile translucide incolore.

| Analyse élémentaire : | | | | |
|---|---|---|---|---|
| | % C | % H | % S | % F |
| Calculé | 40,28 | 4,80 | 5,66 | 43,60 |
| Mesuré | 40,37 | 4,82 | 5,55 | 43,74 |

EXEMPLE II

**1-(2'-F-octyléthylthio)3-(2"-éthylhexyloxy)-2-propanol**

Le composé est préparé de façon analogue à la préparation décrite à l'exemple I où l'on utilise :

- 288 g de 2-F-octyléthanethiol
- 5,4 g d'une solution méthanolique de méthylate de sodium (5,54 meq $g^{-1}$)
- 111,6 g de 2-éthylhexylglycidyléther
- 30 ml de HCl normal

On obtient 337 g (81%) d'une huile translucide incolore.

| Analyse élémentaire : | | | | |
|---|---|---|---|---|
| | % C | % H | % S | % F |
| Calculé | 37,84 | 4,08 | 4,81 | 48,46 |
| Mesuré | 37,83 | 4,06 | 4,20 | 47,45 |

**EXEMPLES DE FORMULATION**

**EXEMPLE 1**

**Fards à paupières**

| | **Parties en poids** |
|---|---|
| **Partie A :** | |
| Mica | 20,0 |
| Dioxyde de titane | 2,0 |
| Stéarate de zinc | 3,0 |
| Poudre de polyamide | 10,0 |
| Oxyde de fer noir | 3,6 |
| Violet de manganèse | 9,6 |
| Talc | 45,1 |
| **Partie B :** | |
| 1-(2'-F-hexyléthylthio)-3-(2"-éthylhexyloxy)-2-propanol | 4,0 |
| Huile de vaseline | 0,5 |
| Huile de ricin | 2,0 |
| Conservateur | 0,2 |
| | 100,0 |

**Mode opératoire** :

On mélange les constituants de la phase A, les constituants de la phase B, puis on ajoute la phase B à la phase A et on mélange à nouveau; on broye éventuellement, on tamise et on compacte dans une coupelle métallique.

Le produit obtenu présente une grande douceur.

**EXEMPLE 2**

**Fards à paupières**

| | Parties en poids |
|---|---|
| **Partie A :** | |
| Oxychlorure de bismuth | 8,0 |
| Stéarate de zinc | 3,0 |
| Mica | 20,0 |
| Oxyde de chlorure | 5,4 |
| Oxydes de fer | 9,1 |
| Talc | 48,3 |
| **Partie B :** | |
| Huile organofluorée hydrocarbonée (NOFABLE-FO 9982, commercialisé par la Société NIPPON OIL & FATS) | 4,5 |
| Huile de jojoba | 1,5 |
| Conservateur | 0,2 |
| | 100,0 |

Le produit, obtenu par le même procédé qu'à l'exemple 1, présente une bonne cohésion et un bon développement des pigments.

**EXEMPLE 3**

**Fards à paupières**

| | Parties en poids |
|---|---|
| **Partie A :** | |
| Oxychlorure de bismuth | 10,0 |
| Mica | 20,0 |
| Dioxyde de titane | 2,0 |
| Oxydes de fer | 15,5 |
| Talc | 46,0 |
| **Partie B :** | |
| 1-(2'-F-hexyléthylthio)-3-(2''-éthylhexyloxy)-2-propanol | 6,5 |
| | 100,0 |

La composition obtenue par le même mode opératoire qu'à l'exemple 1, est très douce et très facile à étaler; elle donne un résultat homogène au maquillage.

**EXEMPLE 4**

**Fards à paupières**

| | Parties en poids |
|---|---|
| **Partie A :** | |
| Oxychlorure de bismuth | 10,0 |
| Mica | 20,0 |
| Stéarate de zinc | 3,0 |
| Oxyde de fer noir | 3,6 |
| Violet de manganèse | 9,6 |
| Bleu d'outremer | 10,8 |
| DC Red 30 | 1,8 |
| Talc | 34,7 |
| **Partie B :** | |
| 1-(2'-F-hexyléthylthio)-3-(2''-éthylhexyloxy)-2-propanol | 6,5 |
| | 100,0 |

Le produit est obtenu selon le même mode opératoire que celui de l'exemple 1.

**EXEMPLE 5**

**Comparatif**

**Fards à paupières**

| | Parties en poids |
|---|---|
| **Partie A :** | |
| Oxychlorure de bismuth | 10,0 |
| Mica | 20,0 |
| Stéarate de zinc | 3,0 |
| Oxyde de fer noir | 3,6 |
| Violet de manganèse | 9,6 |
| Bleu d'outremer | 10,8 |
| DC Red 30 | 1,8 |
| Talc | 34,7 |
| **Partie B :** | |
| Huile perfluoropolyéther (FOMBLIN HC25-MONTEDISON) | 6,5 |
| | 100,0 |

Le produit est obtenu selon le même mode opératoire que celui de l'exemple 1.

L'exemple 4 par rapport à l'exemple 5 présente, en coupelle, une teinte plus intense et sans fond blanc due à un

bon développement des pigments.

Pour obtenir la teinte obtenue dans l'exemple 4, le taux des pigments a dû être augmenté de 29,8% dans l'exemple 5.

**EXEMPLE 6**

**Fards à paupières**

| | Parties en poids |
|---|---|
| **Partie A :** | |
| Oxychlorure de bismuth | 10,0 |
| Mica | 20,0 |
| Stéarate de zinc | 3,0 |
| Oxyde de chrome | 5,4 |
| Oxyde de fer noir | 5,9 |
| Oxyde de fer jaune | 3,2 |
| Talc | 46,0 |
| **Partie B :** | |
| 1-(2'-F-hexyléthylthio)-3-(2''-éthylhexyloxy)-2-propanol | 6,5 |
| | 100,0 |

Le produit est obtenu selon le même mode opératoire que celui de l'exemple 1.

**EXEMPLE 7**

**Comparatif**

**Fards à paupières**

| | Parties en poids |
|---|---|
| **Partie A :** | |
| Oxychlorure de bismuth | 10,0 |
| Mica | 20,0 |
| Stéarate de zinc | 3,0 |
| Oxyde de chrome | 5,4 |
| Oxyde de fer noir | 5,9 |
| Oxyde de fer jaune | 3,2 |
| Talc | 46,0 |
| **Partie B :** | |
| Huile perfluoropolyéther (FOMBLIN HC25-MONTEDISON) | 6,5 |
| | 100,0 |

L'exemple 6 par rapport à l'exemple 7 présente, en coupelle, une teinte beaucoup plus intense et sans fond blanc, due à un bon développement des pigments.

Pour obtenir la teinte obtenue dans l'exemple 6, le taux des pigments a dû être augmenté de 58,6% dans l'exemple 7.

**EXEMPLE 8**

**Fards à joues**

| | **Parties en poids** |
|---|---|
| **Partie A :** | |
| Talc | 70,8 |
| Dioxyde de titane | 5,0 |
| Mica | 10,0 |
| Stéarate de zinc | 2,0 |
| DC Red 30 | 0,5 |
| Oxydes de fer | 6,0 |
| **Partie B :** | |
| 1-(2'-F-hexyléthylthio)-3-(2''-éthylhexyloxy)-2-propanol | 4,5 |
| Huile de ricin | 0,5 |
| Conservateur | 0,2 |
| Parfum | 0,3 |
| | 100,0 |

Le produit est obtenu selon le mode opératoire de l'exemple 1.

**EXEMPLE 9**

**Poudre compacte visage**

| | Parties en poids |
|---|---|
| **Partie A :** | |
| Séricite | 65,8 |
| Mica | 15,0 |
| Poudre de polyéthylène | 5,0 |
| Dioxyde de titane | 2,0 |
| Oxydes de fer | 8,0 |
| **Partie B :** | |
| 1-(2'-F-hexyléthylthio)-3-(2''-éthylhexyloxy)-2-propanol | 5,5 |
| Huile de jojoba | 1,0 |
| Conservateur | 0,2 |
| Parfum | 0,3 |
| | 100,0 |

Le produit, obtenu selon le mode opératoire de l'exemple 1, présente une grande douceur.

**EXEMPLE 10**

**Poudre libre visage**

| | Parties en poids |
|---|---|
| **Partie A :** | |
| Talc | 93,35 |
| Oxyde de fer | 0,7 |
| Dioxyde de titane | 2,0 |
| **Partie B :** | |
| 1-(2'-F-hexyléthylthio)-3-(2''-éthylhexyloxy)-2-propanol | 3,0 |
| Huile de vaseline | 0,75 |
| Parfum | 0,2 |
| | 100,0 |

On mélange les constituants de la partie A que l'on incorpore par mélange aux constituants de la partie B préalablement mélangés, puis on tamise.

**EXEMPLE 11**

**Fards à paupières**

| | **Parties en poids** |
|---|---|
| **Partie A :** | |
| Talc | 58,2 |
| Oxyde de fer jaune | 0,3 |
| Oxyde de fer noir | 0,1 |
| Oxyde de fer brun | 0,2 |
| **Partie B :** | |
| Mica titane | 30 |
| **Partie C :** | |
| 1-(2'-F-hexyléthylthio)-3-(2''-éthylhexyloxy)-2-propanol | 10 |
| Huile de ricin | 1 |
| Conservateur | 0,2 |
| | 100,0 |

**Mode opératoire :**

On mélange les constituants de la phase A, les constituants de la phase C, puis on ajoute la phase C à la phase A et on mélange à nouveau; on broye éventuellement. On ajoute la phase B et on mélange. On tamise et on compacte dans une coupelle métallique.

**EXEMPLE 12**

**Fards à joues**

| | **Parties en poids** |
|---|---|
| **Partie A :** | |
| Dioxyde de titane | 6,0 |
| Stéarate de zinc | 2,0 |
| Mica | 10,0 |
| DC Red 30 | 0,6 |
| Oxydes de fer | 38,0 |
| Talc | 73,1 |
| **Partie B :** | |
| 1-(2'-F-octyléthylthio)-3-(2''-éthylhexyloxy)-2-propanol | 6,5 |
| | 100,0 |

Le produit est obtenu selon le mode opératoire de l'exemple 1.

## Revendications

1. Utilisation d'au moins un composé organofluoré hydrocarboné comme liant dans une composition cosmétique sous forme de poudre, les composés organofluorés hydrocarbonés ayant un taux de substitution des atomes d'hydrogène liés aux atomes de carbone par des atomes de fluor, compris entre 0,5 et 95%.

2. Utilisation selon la revendication 1, caractérisée en ce que la composition comporte 0,1 à 35% en poids d'une phase grasse et 99,9 à 65% en poids d'une phase particulaire comportant pigments et/ou charges.

3. Utilisation selon la revendication 1 ou 2, caractérisée en ce que les composés organofluorés hydrocarbonés ont un taux de substitution des atomes d'hydrogène liés aux atomes de carbone par des atomes de fluor, compris entre 10 et 80%.

4. Utilisation selon l'une des revendications 1 à 3, caractérisée en ce que dans la composition cosmétique sous forme de poudre, la concentration dans lesdits composés organofluorés hydrocarbonés est comprise entre 0,1 et 35% en poids par rapport au poids total de la composition.

5. Utilisation selon l'une des revendications 1 à 4, caractérisée en ce que lesdits composés organofluorés hydrocarbonés ont pour formule :

$$(R_F)_x - (A)_y - (R_H)_z \qquad (I)$$

dans laquelle

x représente , 2 ou 3,
y représente 0 ou 1,
z représente 0, 1, 2 ou 3,
à la condition que y et z ne soient pas simultanément nuls et que lorsque z est 0, x est 2 ou 3,
$R_F$ représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,
$R_H$ représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote,
A représente un radical di, tri ou tétravalent tel que
$>C<$ , $>CH-$, $-N<$ , $-CO-N<$ , $-SO_2N<$ ,

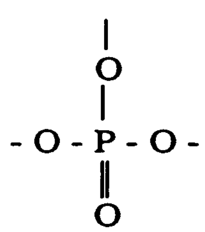

les structures cycliques, aliphatiques ou aromatiques ou les insaturations éthyléniques.

6. Utilisation selon l'une des revendications 1 à 5, caractérisée en ce que lesdits composés organofluorés hydrocarbonés ont pour formule :

$$R_1 - (CH_2)_n - X - [C_3H_5(OH)] - (Y)_x - R_2 \qquad (II)$$

dans laquelle $C_3H_5(OH)$ représente les structures :

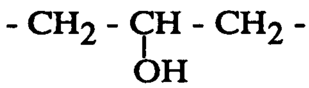 ou 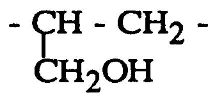

(Ia) (Ib)

$R_1$ représente un radical alkyle perfluoré en $C_4$-$C_{20}$ ou un mélange de radicaux alkyle perfluorés en $C_4$-$C_{20}$;
$R_2$ représente un radical alkyle linéaire ou ramifié en $C_1$-$C_{22}$ ou un mélange de radicaux alkyle linéaires ou ramifiés en $C_1$-$C_{22}$ ou un radical aryle en $C_6$-$C_{10}$ ou aralkyle en $C_7$-$C_{15}$;
n est compris entre 0 et 4;
X et Y représentent O, S,

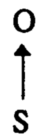 ou 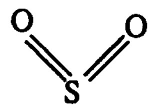

x représente O ou 1 ;
sous réserve que lorsque X est S,

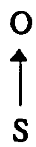 ou 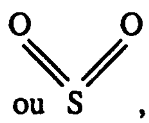 ,

Y représente O .

7. Utilisation selon l'une des revendications 2 à 6, caractérisée en ce que la phase grasse comporte, outre le composé organofluoré hydrocarboné, au moins une huile ou cire hydrocarbonée ou siliconée.

8. Composition cosmétique sous forme de poudre, constituée d'une phase particulaire comportant pigments et/ou charges et d'une phase grasse, caractérisée en ce qu'elle comporte 1,01 à 35% en poids d'au moins un composé organofluoré hydrocarboné ayant un taux de substitution des atomes d'hydrogène liés aux atomes de carbone par des atomes de fluor, compris entre 0,5 et 95% et 98,99 à 65% en poids d'une phase particulaire comportant pigments ou charges.

9. Composition selon la revendication 8, caractérisée en ce que le composé organofluoré hydrocarboné a un taux de substitution compris entre 10 et 80%.

10. Composition selon la revendication 8 ou 9, caractérisée en ce que le composé organofluoré hydrocarboné est de formule (I) :

$$(R_F)_x - (A)_y - (R_H)_z \qquad (I)$$

dans laquelle :

x représente 1, 2 ou 3,
y représente 0 ou 1,
z représente 0, 1, 2 ou 3,

à la condition que y et z ne soient pas simultanément nuls et que lorsque z est 0, x est 2 ou 3,

$R_F$ représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote et/ou substituée par des atomes d'hydrogène ou d'autres atomes d'halogène, à la condition que, pour deux atomes de carbone du squelette, ne soit pas présent plus d'un de ces substituants autres que le fluor,

$R_H$ représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote,

A représente un radical di, tri ou tétradrivalent tel que

$>C<$ , $>CH-$ , $-N<$ , $-CO-N<$, $-SO_2N<$ ,

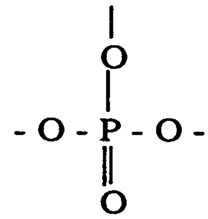

les structures cycliques, aliphatiques ou aromatiques ou les insaturations éthyléniques.

**11.** Composition cosmétique selon l'une des revendications 8 à 10, caractérisée en ce que le composé organofluoré hydrocarboné a la formule (II) suivante :

$$R_1 - (CH_2)_n - X - [C_3H_5(OH)] - (Y)_x - R_2 \qquad (II)$$

dans laquelle $C_3H_5(OH)$ représente les structures :

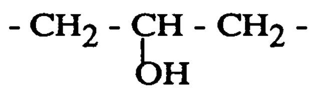 ou 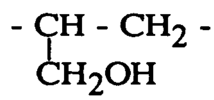

(Ia) (Ib)

$R_1$ représente un radical alkyle perfluoré en $C_4$-$C_{20}$ ou un mélange de radicaux alkyle perfluorés en $C_4$-$C_{20}$;

$R_2$ représente un radical alkyle linéaire ou ramifié en $C_1$-$C_{22}$ ou un mélange de radicaux alkyle linéaires ou ramifiés en $C_1$-$C_{22}$ ou un radical aryle en $C_6$-$C_{10}$ ou aralkyle en $C_7$-$C_{15}$;

n est compris entre 0 et 4 ;

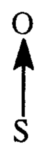 ou 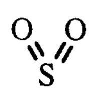

X et Y représentent O, S,

x représente O ou 1 ;
sous réserve que lorsque X est S,

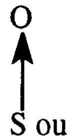 ou 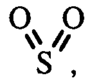 ,

Y représente O.

**12.** Composition cosmétique selon l'une des revendications 8 à 11, caractérisée en ce qu'elle comporte de 3 à 15 % en poids par rapport au poids total de la composition du composé organofluoré hydrocarboné.

**13.** Composition cosmétique selon l'une des revendications 8 à 12, carcactérisée en ce que la phase grasse représente 1,01 à 35 % du poids total de la composition, de préférence 3 à 15 % en poids.

**14.** Composition selon l'une des revendications 8 à 13, caractérisée en ce que la phase grasse comporte, outre le composé organofluoré hydrocarboné, au moins une huile ou une cire hydrocarbonée ou siliconée.

**15.** Composition selon l'une des revendications 8 à 14, caractérisée en ce qu'elle est sous forme de fards à paupières, fards à joues, poudre compacte ou libre pour le maquillage du visage ou de poudre corporelle.

**16.** Utilisation selon la revendication 6, caractérisée en ce que lesdits composés organofluorés hydrocarbonés sont le 1-(2'-F-hexyléthylthio)-3-(2''-éthylhexyloxy)-2-propanol et le 1-(2'-F-octyléthylthio)-3-(2''-éthylhexyloxy)-2-propanol.

**17.** Composition selon la revendication 8, caractérisée en ce que lesdits composés organofluorés hydrocarbonés sont le 1-(2'-F-hexyléthylthio)-3-(2''-éthylhexyloxy)-2-propanol et le 1-(2'-F-octyléthylthio)-3-(2''-éthylhexyloxy)-2-propanol.

**Claims**

**1.** Use of at least one organofluorine hydrocarbon compound as a binder in a cosmetic powder composition, the organofluorine hydrocarbon compounds having a substitution ratio of hydrogen atoms bonded to carbon atoms by fluorine atoms of between 0.5% and 95%.

**2.** Use according to claim 1 characterised in that the composition comprises 0.1% to 35% by weight of a greasy phase and 99.9% to 65% by weight of a particulate phase comprising pigments and/or fillers.

**3.** Use according to claim 1 or claim 2 characterised in that the organofluorine hydrocarbon compounds have a substitution ratio of hydrogen atoms bonded to carbon atoms by fluorine atoms of between 10% and 80%.

**4.** Use according to any one of claims 1 to 3 characterised in that, in the cosmetic powder composition, the concentration of said organofluorine hydrocarbon compounds is between 0.1% and 35% by weight with respect to the total composition weight.

**5.** Use according to any one of claims 1 to 4 characterised in that said organofluorine hydrocarbon compounds have formula:

$$(R_F)_x - (A)_y - (R_H)_z \qquad (I)$$

where

$\underline{x}$ represents 1, 2 or 3,
$\underline{y}$ represents 0 or 1,
$\underline{z}$ represents 0, 1, 2 or 3,

provided that y and z are not simultaneously 0 and that when z is 0, x is 2 or 3,

$R_F$ represents an aliphatic or aromatic, saturated or unsaturated, linear, branched or cyclic fluorinated radical, in which the chain may be functionalised and/or interrupted by divalent atoms such as oxygen or sulphur or trivalent atoms such as nitrogen and/or substituted by hydrogen atoms or other halogen atoms, provided that, for any two carbon atoms of the backbone, no more than one of these substituents other than fluorine is present,

$R_H$ represents an aliphatic or aromatic, saturated or unsaturated, linear, branched or cyclic hydrocarbon radical, in which the chain may be functionalised and/or interrupted by one or more divalent atoms such as oxygen or sulphur or one or more trivalent atoms such as nitrogen,

A represents a di-, tri- or tetravalent radical such as

$>C<$, $>CH-$, $-N<$, $-CO-N<$, $-SO_2N<$,

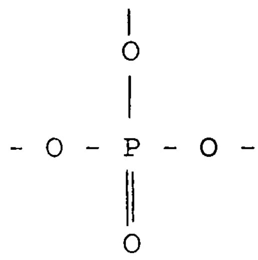

or cyclic, aliphatic or aromatic structures, or ethylenic unsaturations.

6. Use according to any one of claims 1 to 5 characterised in that said organofluorine hydrocarbon compounds have formula:

$$R_1 - (CH_2)_n - X - [C_3H_5(OH)] - (Y)_x - R_2 \qquad \text{(II)}$$

where

$C_3H_5(OH)$ represents the structures:

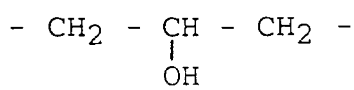 or 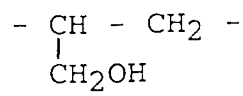

(Ia) (Ib)

$R_1$ represents a perfluorine $C_4$-$C_{20}$ alkyl radical or a mixture of perfluorine $C_4$-$C_{20}$ alkyl radicals;

$R_2$ represents a linear or branched $C_1$-$C_{22}$ alkyl radical or a mixture of linear or branched $C_1$-$C_{22}$ alkyl radicals, a $C_6$-$C_{10}$ aryl radical or a $C_7$-$C_{15}$ aralkyl radical;

n is between 0 and 4;

X and Y represent O, S,

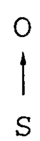 or 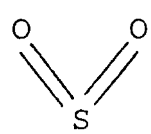

x represents 0 or 1;

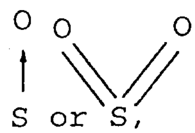

provided that, when X is S, Y represents O.

7. Use according to any one of claims 2 to 6 characterised in that the greasy phase comprises, apart from the organofluorine hydrocarbon compound, at least one hydrocarbon or silicone oil or wax.

8. Cosmetic powder composition constituted by a particulate phase comprising pigments and/or fillers and a greasy phase characterised in that it comprises 1.01% to 35% by weight of at least one organofluorine hydrocarbon compound having a ratio of substitution of hydrogen atoms bonded to carbon atoms by fluorine atoms of between 0.5% and 95%, and 98.99% to 65% by weight of a particulate phase comprising pigments or fillers.

9. Composition according to claim 8 characterised in that the organofluorine hydrocarbon compound has a substitution ratio of between 10% and 80%.

10. Composition according to claim 8 or claim 9, characterised in that the organofluorine hydrocarbon compound has formula (I):

$$(R_F)_x - (A)_y - (R_H)_z \qquad (I)$$

where:

<u>x</u> represents 1, 2 or 3,
<u>y</u> represents 0 or 1,
<u>z</u> represents 0, 1, 2 or 3,
provided that <u>y</u> and <u>z</u> are not simultaneously 0 and that when <u>z</u> is 0, <u>x</u> is 2 or 3,
$R_F$ represents an aliphatic or aromatic, saturated or unsaturated, linear, branched or cyclic fluorinated radical, in which the chain may be functionalised and/or interrupted by divalent atoms such as oxygen or sulphur or trivalent atoms such as nitrogen and/or substituted by hydrogen atoms or other halogen atoms, provided that, for any two carbon atoms of the backbone, no more than one of these substituents other than fluorine is present,
$R_H$ represents an aliphatic or aromatic, saturated or unsaturated, linear, branched or cyclic hydrocarbon radical, in which the chain may be functionalised and/or interrupted by one or more divalent atoms such as oxygen or sulphur or one or more trivalent atoms such as nitrogen,
A represents a di-, tri- or tetravalent radical such as
$>C<$, $>CH-$, $-N<$, $-CO-N<$, $-SO_2N<$,

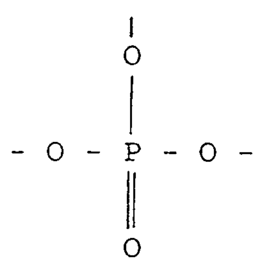

or cyclic, aliphatic or aromatic structures, or ethylenic unsaturations.

11. Cosmetic composition according to any one of claims 8 to 10 characterised in that the organofluorine hydrocarbon compound has formula (II):

$$R_1 - (CH_2)_n - X - [C_3H_5(OH)] - (Y)_x - R_2 \qquad (II)$$

where

$C_3H_5(OH)$ represents the structures:

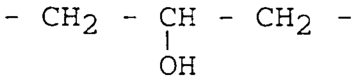

(Ia)

or

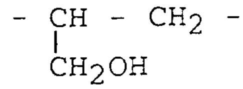

(Ib)

$R_1$ represents a perfluorine $C_4$-$C_{20}$ alkyl radical or a mixture of perfluorine $C_4$-$C_{20}$ alkyl radicals;
$R_2$ represents a linear or branched $C_1$-$C_{22}$ alkyl radical or a mixture of linear or branched $C_1$-$C_{22}$ alkyl radicals, a $C_6$-$C_{10}$ aryl radical or a $C_7$-$C_{15}$ aralkyl radical;
n is between 0 and 4;

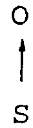

or

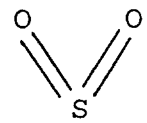

X and Y represent O, S,
x represents 0 or 1;

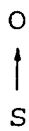

or

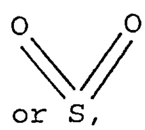

,

provided that, when X is S, Y represents O.

**12.** Cosmetic composition according to any one of claims 8 to 11 characterised in that it comprises 3% to 15% by weight of organofluorine hydrocarbon compound with respect to the total composition weight.

**13.** Cosmetic composition according to any one of claims 8 to 12 characterised in that the greasy phase represents 1.01% to 35% of the total composition weight, preferably 3% to 15% by weight.

**14.** Cosmetic composition according to any one of claims 8 to 13 characterised in that, apart from the organofluorine hydrocarbon compound, the greasy phase comprises at least one hydrocarbon or silicone oil or wax.

**15.** Cosmetic composition according to any one of claims 8 to 14 characterised in that it is in the form of an eyeshadow, blusher or compressed or loose face or body powder.

**16.** Use according to claim 6 characterised in that said organofluorine hydrocarbon compounds are 1-(2'-F-hexylethylthio)-3-(2''-ethylhexyloxy)-2-propanol and 1-(2'-F-octylethylthio)-3-(2''-ethylhexyloxy)-2-propanol.

**17.** Composition according to claim 8 characterised in that said organofluorine hydrocarbon compounds are 1-(2'-F-hexylethylthio)-3-(2''-ethylhexyloxy)-2-propanol and 1-(2'-F-octylethylthio)-3-(2''-ethylhexyloxy)-2-propanol.

**Patentansprüche**

**1.** Verwendung mindestens einer Organofluorkohlenwasserstoffverbindung in einer in Pulverform vorleigenden kosmetischen Zusammensetzung, wobei die Organofluorkohlenwasserstoffverbindungen einen Substitutionsgehalt,

der an die Kohlenstoffatome gebundene Wasserstoffatome durch die Fluoratome von 0,5 bis 95% aufweisen.

**2.** Verwendung gemäß Anspruch 1,
dadurch **gekennzeichnet**, daß

die Zusammensetzung 0,1 bis 35 Gew.% Fettphase und 99,9 bis 65 Gew.% einer teilchenförmigen Phase umfaßt, die Pigmente und/oder Beaufschlagungsmittel enthält.

**3.** Verwendung gemäß Anspruch 1 oder 2,
dadurch **gekennzeichnet**, daß

die Organofluorkohlenwasserstoffverbindungen einen Substitutionsgehalt der an die Kohlenstoffatome gebundenen Wasserstoffatome durch die Fluoratome von 10 bis 80% aufweisen.

**4.** Verwendung gemäß einem der Ansprüche 1 bis 3,
dadurch **gekennzeichnet**, daß

in der in Pulverform vorliegenden kosmetischen Zusammensetzung die Konzentration der genannten Organofluorkohlenwasserstoffverbindungen 0,1 bis 35 Gew.% beträgt, bezogen auf das Gesamtgewicht der Zusammensetzung.

**5.** Verwendung gemäß einem der Ansprüche 1 bis 4,
dadurch **gekennzeichnet**, daß

die genannten Organofluorkohlenwasserstoffverbindungen die Formel aufweisen:

$$(R_F)_x - (A)_y - (R_H)_z \qquad (I)$$

worin gilt:
x ist 1, 2 oder 3,
y ist 0 oder 1,
z ist 0, 1, 2 oder 3,
mit der Maßgabe, daß y und z nicht gleichzeitig 0 sind, und daß, wenn Z 0 ist, x 2 oder 3 ist,
$R_F$ stellt einen aliphatischen oder aromatischen, gesättigten oder ungesättigten fluorhaltigen Rest mit linearer, verzweigter oder zyklischer Kette dar, wobei diese Kette mit zweiwertigen Atomen wie Sauerstoff oder Schwefel oder mit dreiwertigen wie Stickstoff funktionalisiert und/oder unterbrochen und/oder mit Wasserstoffatomen oder anderen Halogenatomen substituiert sein kann, mit der Maßgabe, daß, für zwei Kohlenstoffatome des Gerüsts, nicht mehr als einer dieser Substituenten vorhanden ist, die sich von Fluor unterscheiden,
$R_H$ stellt einen aliphatischen oder aromatischen, gesättigten oder ungesättigten Kohlenwasserstoffrest mit linearer, verzweigter oder zyklischer Kette dar, wobei diese Kette mit einem oder mehreren zweiwertigen Atomen wie Sauerstoff oder Schwefel oder mit einem oder mehreren dreiwertigen Atomen wie Stickstoff funktionalisiert und/oder unterbrochen ist, A stellt einen zwei-, drei- oder vierwertigen Rest wie:
$>C<$, $>CH-$, $-N<$, $-CO-N<$, $-SO_2N<$,

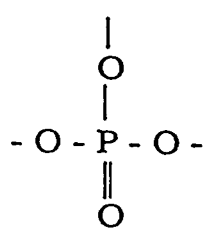

zyklische, aliphatische oder aromatische Strukturen oder ethylenisch ungesättigte Gruppen dar.

**6.** Verwendung gemäß einem der Ansprüche 1 bis 5,
dadurch **gekennzeichnet**, daß

die genannten Organofluorkohlenwasserstoffverbindungen die Formel aufweisen:

$$R_1 - (CH_2)_n - X - [C_3H_5(OH)] - (Y)_x - R_2 \quad (II)$$

worin $C_3H_5(OH)$ die Strukturen darstellt:

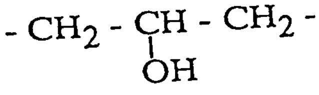
(Ia)

oder

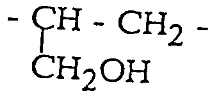
(Ib)

worin gilt:
$R_1$ stellt einen $C_{4-20}$-Perfluoralkylrest oder eine Mischung aus $C_{4-20}$-Perfluoralkylresten dar;
$R_2$ stellt einen linearen oder verzweigten $C_{1-22}$-Alkylrest oder eine Mischung aus linearen oder verzweigten $C_{1-22}$-Alkylresten oder einen $C_{6-10}$-Aryl- oder $C_{7-15}$-Aralkylrest dar;
n beträgt 0 bis 4;
X und Y stellen O, S,

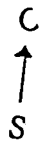

oder

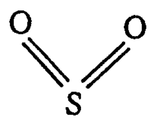

dar;
x beträgt 0 oder 1; mit der Maßgabe, daß wenn X S,

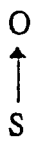

oder

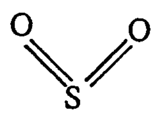

ist, Y O darstellt.

7. Verwendung gemäß einem der Ansprüche 2 bis 6,
dadurch **gekennzeichnet**, daß

die Fettphase, ausser der Organofluorkohlenwasserstoffverbindung, mindestens ein Kohlenswasserstoff- oder Siliconöl oder ein entsprechendes Wachs enthält.

8. Kosmetische Zusammensetzung in Pulverform, die aus einer teilchenförmigen Phase, enthaltend Pigmente und/oder Beaufschlagungsmittel, und einer Fettphase zusammengesetzt ist,
dadurch **gekennzeichnet**, daß

sie 1,01 bis 35 Gew.% mindestens einer Organofluorkohlenwasserstoffverbindung, die einen Substitutionsgehalt der an die Kohlenstoffatme gebundenen Wasserstoffatome durch die Fluoratome von 0,5 bis 95% aufweist, sowie 98,99 bis 65 Gew.% einer teilchenförmigen Phase, enthaltend Pigmente oder Beaufschlagungsmittel, umfaßt.

9. Zusammensetzung gemäß Anspruch 8,
dadurch **gekennzeichnet**, daß

die Organofluorkohlenwasserstoffverbindung einen Substitutionsgehalt von 10 bis 80% aufweist.

**10.** Zusammensetzung gemäß Anspruch 8 oder 9,
dadurch **gekennzeichnet**, daß

die Organofluorkohlenwasserstoffverbindung die Formel (I) aufweist:

$$(R_F)_x - (A)_y - (R_H)_z \qquad (I)$$

worin gilt:
x ist 1, 2 oder 3,
y ist 0 oder 1,
z ist 0, 1, 2 oder 3,
mit der Maßgabe, daß y und z nicht gleichzeitig 0 sind, und daß, wenn Z 0 ist, x 2 oder 3 ist,
$R_F$ stellt einen aliphatischen oder aromatischen, gesättigten oder ungesättigten fluorhaltigen Rest mit linearer, verzweigter oder zykilischer Kette dar, wobei diese Kette mit zweiwertigen Atomen wie Sauerstoff oder Schwefel oder mit dreiwertigen wie Stickstoff funktionalisiert und/oder unterbrochen und/oder mit Wasserstoffatomen oder anderen Halogenatomen substituiert sein kann, mit der Maßgabe, daß, für zwei Kohlenstoffatome des Gerüsts, nicht mehr als einer dieser Substituenten vorhanden ist, die sich von Fluor unterscheiden,
$R_H$ stellt einen aliphatischen oder aromatischen, gesättigten oder ungesättigten Kohlenwasserstoffrest mit linearer, verzweigter oder zyklischer Kette dar, wobei diese Kette mit einem oder mehreren zweiwertigen Atomen wie Sauerstoff oder Schwefel oder mit einem oder mehreren dreiwertigen Atomen wie Stickstoff funktionalisiert und/oder unterbrochen ist,
A stellt einen zwei-, drei- oder vierwertigen Rest wie:
$>C<$, $>CH-$, $-N<$, $-CO-N<$, $-SO_2N<$,

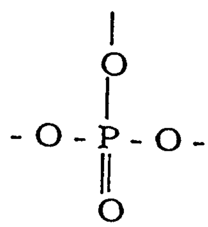

zyklische, aliphatische oder aromatische Strukturen oder ethylenisch ungesättigte Gruppen dar.

**11.** Kosmetische Zusammensetzung gemäß einem der Ansprüche 8 bis 10,
dadurch **gekennzeichnet**, daß

die Organofluorkohlenwasserstoffverbindung die folgende Formel (II) aufweist:

$$R_1 - (CH_2)_n - X - [C_3H_5(OH)] - (Y)_x - R_2 \qquad (II)$$

worin $C_3H_5(OH)$ die Strukturen darstellt:

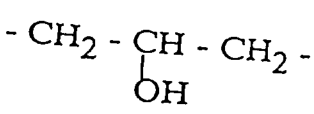
(Ia)
oder
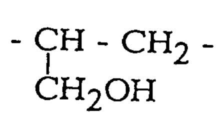
(Ib)

worin gilt:
$R_1$ stellt einen $C_{4-20}$-Perfluoralkylrest oder eine Mischung aus $C_{4-20}$-Perfluoralkylresten dar;
$R_2$ stellt einen linearen oder verzweigten $C_{1-22}$-Alkylrest oder eine Mischung aus linearen oder verzweigten $C_{1-}$

$_{22}$-Alkylresten oder einen $C_{6-10}$-Aryl- oder $C_{7-15}$-Aralkylrest dar;
n beträgt 0 bis 4;
X und Y stellen O, S,

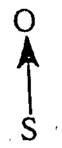 oder 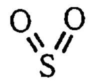

dar;
x beträgt 0 oder 1;
mit der Maßgabe, daß, wenn X S,

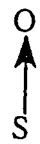 oder 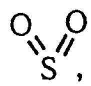,

ist, Y O darstellt.

**12.** Kosmetische Zusammensetzung gemäß einem der Ansprüche 8 bis 11,
dadurch **gekennzeichnet**, daß

sie 3 bis 15 Gew.% Organofluorkohlenwasserstoffverbindung enthält, bezogen auf das Gesamtgewicht der Zusammensetzung.

**13.** Kosmetische Zusammensetzung gemäß einem der Ansprüche 8 bis 12,
dadurch **gekennzeichnet**, daß

die Fettphase 1,01 bis 35 und vorzugsweise 3 bis 15 Gew.% ausmacht, bezogen auf das Gesamtgewicht der Zusammensetzung.

**14.** Zusmamensetzung gemäß einem der Ansprüche 8 bis 13,
dadurch **gekennzeichnet**, daß

die Fettphase, ausser der Organofluorkohlenwasserstoffverbindung, mindestens ein Kohlenwasserstoff- oder Siliconöl oder ein entsprechendes Wachs enthält.

**15.** Zusammensetzung gemäß einem der Ansprüche 8 bis 14,
dadurch **gekennzeichnet**, daß

sie in Form von Lidschatten, Wangenschminken, kompaktem oder freien Puder zum Schminken des Gesichts oder von Körperpuder vorliegt.

**16.** Verwendung gemäß Anspruch 6,
dadurch **gekennzeichnet**, daß

die genannten Organofluorkohlenwasserstoffverbindungen 1-(2'-F-Hexylethylthio)-3-(2"-ethylhexyloxy)propan-2-ol und 1-(2'-F-Octylethylthio)-3-(2"-ethylhexyloxy)propan-2-ol sind.

**17.** Zusammensetzung gemäß Anspruch 8,
dadurch **gekennzeichnet**, daß

die genannten Organofluorkohlenwasserstoffverbindungen 1-(2'-F-Hexylethylthio)-3-(2"-ethylhexyloxy)propan-2-ol und 1-(2'-F-Octylethylthio)-3-(2"-ethylhexyloxy)propan-2-ol sind.